# DEMANDE DE BREVET EUROPEEN

(11) **EP 1 138 773 A2**
(43) Date de publication de la demande: **04.10.2001**
(21) Numéro de dépôt: 01102630.9
(22) Date de dépôt: 07.04.1997
(51) Int. Cl.: C12N 15/86, C12N 7/01, C12N 7/04, A61K 39/21, C07K 14/16

(54) **Utilisation de plasmides pour la fabrication d'une composition destinée à la mise en oeuvre d'une méthode de traitement vaccinal chez l'homme ou l'animal**

(30) Priorité: 05.04.1996 FR 9604370
(62) Demande divisionnaire de: 97400796.5
(71) Demandeur: UNIVERSITE PIERRE ET MARIE CURIE PARIS VI, 75252 Paris Cédex 05 (FR)
(72) Inventeur: Klatzmann, David, 75013 Paris (FR); Salzmann, Jean-Loup, 75005 Paris (FR)
(74) Mandataire: Becker, Philippe

(57) **Abrégé**

La présente invention se rapporte à un vaccin constitué de particules virales défectives telles qu'obtenues in vivo ou ex vivo chez des individus infectés ou susceptibles d'être infectés par un virus, après expression des gènes portés par un vecteur ou une association de vecteurs et comprenant au moins les gènes de structure nécessaires à la constitution de la particule virale.

## Description

La présente invention porte sur des vaccins préventifs ou thérapeutiques constitués de particules virales telles qu'obtenues in vivo après l'expression de gènes portés par un plasmovirus.

Un plasmovirus est défini comme un plasmide ou un vecteur viral, porteur de l'ensemble des séquences nécessaires à la reconstitution d'une particule virale après expression dans une cellule hôte, in vivo, in vitro ou ex vivo. Autrement dit, l'introduction du plasmovirus dans la cellule hôte transforme cette dernière en cellule d'encapsidation productrice de virions.

La vaccination contre des virus et en particulier des rétrovirus est une opération complexe. Quelques stratégies se sont révélées plus ou moins efficaces pour la vaccination contre les oncovirus, mais les essais de vaccins contre des lentivirus ne donnent pas de résultats satisfaisant. Une des raisons qui pourrait expliquer cet état de fait est que l'ensemble des phénomènes conduisant à une immunisation contre un virus nécessite probablement l'existence de particules virales physiques et la production de ces particules par des cellules de l'hôte. Une meilleure preuve en est que les meilleurs résultats de vaccination des singes contre le virus SIV l'ont été par l'utilisation de virus atténués se répliquant chez les singes vaccinés. Une stratégie de vaccination thérapeutique a été explorée par Salk et al, (Proc. Natl. Acad. Sci. USA (1991) 88 : 3348-52) en utilisant des particules HIV inactivées. Cette stratégie s'est révélée insatisfaisante pour deux raisons. La première est qu'il est difficile de produire du virus HIV in vitro en quantité suffisante et la deuxième est que les procédés d'inactivation desdits virus pour être efficaces détruisaient une partie des épitopes immunogènes des particules virales.

L'objet de la présente invention est de faire produire par la cellule de l'hôte des particules virales reproduisant au plus près les particules virales sauvages mais ou atténués ou incapables de se répliquer chez l'hôte.

Cette approche de vaccination consistant à injecter un plasmovirus, dont l'expression aboutit à la formation de particules virales, est particulièrement intéressante dans le cas où la vaccination par des sous-unités virales n'a pu encore aboutir. L'idée contenue dans cette approche est qu'il n'y a pas de meilleure immunisation que celle obtenue avec une particule physique reproduisant la particule infectieuse. C'est d'ailleurs pour cela que la vaccination contre de nombreux agents pathogènes et virus en particulier, se fait à l'aide de ces virus sous forme atténuée. En particulier, dans le cas de l'infection par le VIH, tous les essais actuels montrent que la seule manière de protéger efficacement un macaque d'une infection par un SIV est l'administration d'une souche atténuée qui protége effectivement d'un challenge contre un virus différent et pathogène. Une telle approche qui est très discutable pour la vaccination de l'homme contre le HIV se heurte aux différents problèmes d'utilisation d'un HIV inactivé qui, par inactivation imparfaite ou par des transcomplémentations ou mutations, pourrait redevenir virulent. Dans l'hypothèse de la construction d'un plasmovirus fait sur une charpente HIV, et dépourvu d'un certain nombre de gènes, hormis ceux permettant de reconstituer la particule physique, l'injection d'un tel plasmide devrait aboutir à la fabrication par les cellules de toutes les protéines nécessaires à la fabrication des particules virales dépourvues d'un génome infectieux ou mieux vides. Cette approche peut permettre de produire in vivo un virus non pas atténué, mais défectif.

L'innoculation de gènes a été montrée comme pouvant générer une réponse immunitaire après injection dans la souris (B. Wang et al. dans Proc.Nat.Acad.Sci.USA 90:4156, (1993) et Ulmer et al, Science, 1993, 259 1745-1749) mais la seule réponse décrite y est la production d'anticorps.

La présente invention est relative à un nouveau moyen de vaccination qui, constitué de particules virales physiques, soit vides soit contenant un génome viral, défectif atténué ou non, et tel qu'obtenu in vivo chez des individus humains ou animaux infectés ou susceptibles d'être infectés par un virus, après expression d'un plasmovirus. Ledit plasmovirus est un vecteur comprenant les gènes de structure nécessaires à la constitution de ladite particule virale.

Par génome viral atténué, on entend tout génome viral modifié de telle façon que la réplication soit peu efficace, par exemple une modification d'un gène qui ne permettrait l'infection que par contact et non par dissémination, par exemple une délétion de la partie extracellulaire du gène env pour ne garder que la partie transmembranaire, fusogénique.

Toute modification du génome viral susceptible de conférer au virus cette caractéristique d'atténuation peut être qualifiée par un test d'infectivité en culture cellulaire où, par des techniques classiques, le taux de virus infectieux présents dans le surnageant de culture est considérablement réduit alors que l'infection de cellule à cellule est peu affectée par cette modification.

Le concept de plasmovirus permettant une reconstitution de particules virales peut être mis en oeuvre de différentes manières selon les propriétés que l'on souhaite conférer au produit pharmaceutique final.

Différents modes de réalisation du plasmovirus sont envisageables, à partir du moment où sur un même plasmide ou vecteur, ou sur plusieurs plasmides ou vecteurs associés, l'ensemble des séquences qui, après avoir pénétré sous forme d'ADN ou d'ARN dans les cellules et s'y être exprimé, reconstitue une particule virale physique.

Par plasmides ou vecteurs associés, on entend des plasmides, vecteur ou séquences d'acides nucléiques dont l'expression est sensiblement simultanée dans les cellules et sont : soit non liés, soit couplés directement ou indirectement, de manière covalente ou non.

Les modes de réalisation sont alors choisis selon la fonction que l'on souhaite conférer au virion reconstitué à partir de la construction plasmovirale.

A titre d'exemple, quand la charpente nucléocapsidique du virus est celle d'un rétrovirus, on cherche à reproduire au sein de l'organisme une situation semblable à celle de l'infection physiologique par le virus en ce sens qu'il y a d'une part une cellule de l'organisme produisant les particules rétrovirales et présentant donc à sa surface des antigènes spécifiques du rétrovirus, et d'autre part sont relarguées dans le milieu extérieur à cette cellule des particules rétrovirales exactement semblables à celles du virus HIV à la seule différence que ces particules sont atténuées ou incapables de se répliquer. L'ensemble des éléments permettant d'obtenir une réponse immune adéquate au virus considéré sont donc présents simultanément dans l'organisme.

Les modes de réalisation sont divers et peuvent privilégier soit la sécurité, soit l'efficacité, soit la sélectivité vis-à-vis d'une cible, soit l'expression d'un transgène particulier, soit plusieurs de ces fonctions simultanément.

Le vecteur peut notamment contenir le gène gag ou les gènes gag et env ou enfin les gènes gag, pol et env du rétrovirus, ces gènes étant éventuellement sous le contrôle de promoteurs indépendants et/ou spécifiques, ainsi que des transgènes d'intérêt sous le contrôle de l'un ou l'autre promoteur ou d'un promoteur indépendant. Un promoteur est dit spécifique ou indépendant s'il est différent du promoteur du virus que l'on reconstitue à partir du plasmovirus, par exemple d'un LTR.

Un mode de réalisation d'un vaccin selon l'invention est celui d'un vaccin contre un rétrovirus, par exemple un HIV et consistant à faire exprimer par les cellules de l'hôte des particules rétrovirales portant les protéines d'enveloppe du virus mais dépourvues du génome viral.

Dans un autre mode de réalisation, la cellule de l'hôte est transduite par un plasmovirus comportant l'ensemble des séquences nécessaires à la fabrication par la cellule d'un virus (séquence gag, pol et env et notamment celles du virus HIV1 ou/et HIV2) mais dépourvu de la séquence Ψ d'encapsidation, le gène pol comportant le cas échéant des mutations dans INT et//ou dans RT. ce qui conduit à la reconstitution dans la cellule cible d'une particule vide ou porteuse de génome défectif.

Dans un autre mode de réalisation, la cellule de l'hôte est transduite par un plasmovirus comportant les séquences gag, pol et pourvue d'un gène env muté ou déficient.

Un autre mode de réalisation est celui où le plasmovirus est pourvu de la séquence d'encapsidation mais dépourvu de certains gènes notamment ceux de la réverse transcriptase et/ou de l'intégrase enlevant à ce dernier toute capacité à se répliquer et/ou à se propager dans l'organisme.

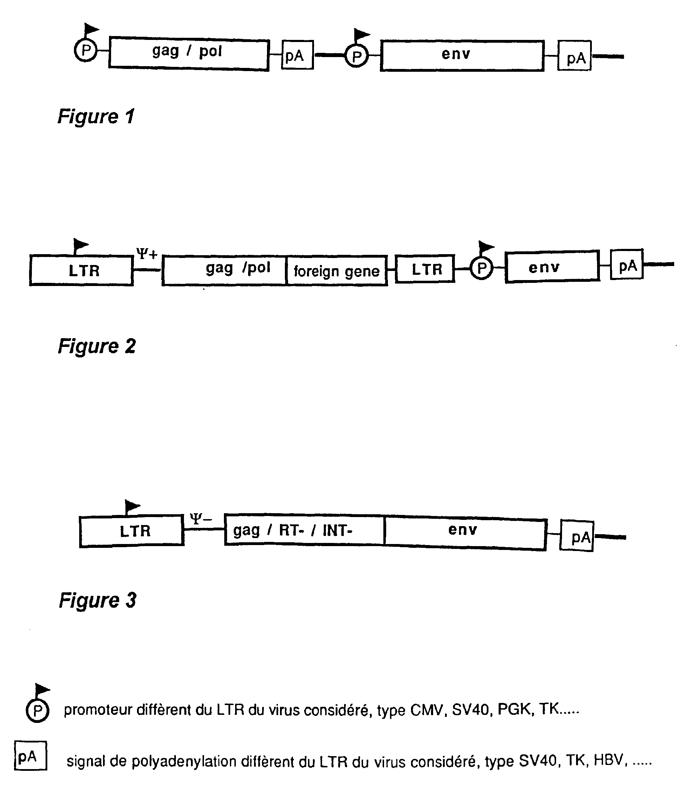

Des exemples non limitatifs de ce type de constructions sont illustrés dans les figures 1 à 3.

Il peut être avantageux d'insérer un gène dans la construction, et dont l'expression présente un intérêt soit :
- pour le traitement vaccinal lui-même pour augmenter la réponse immunogène par exemple en favorisant le déclenchement d'une réaction de type cellulaire et humorale simultanément ;
- pour augmenter la sécurité du système ;
- pour permettre de développer une réaction immunitaire contre un épitope ou un antigène pour lequel les systèmes classiques fournissent des résultats décevants.

Le gène peut être inséré, soit sous le contrôle des promoteurs de gag/pol, soit sous le contrôle du promoteur de env. Si son expression est sous le contrôle d' un LTR de rétrovirus, et que la charpente nucléocapsidique contient la séquence ψ, ce gène sera réintégré dans les virions formés après transfection des cellules cibles, et son expression pourra être poursuivie après la formation du virion *in situ.*

Des exemples de gènes dont l'expression présente un intérêt associé au traitement vaccinal, ou lié à la sécurité du système sont les suivants :
a) un gène codant une toxine conditionnelle, par exemple celui de la thymidine kinase ou un équivalent fonctionnel : il est aujourd'hui bien connu (voir par exemple WO 95/22617) que l'expression de ce gène en des cellules en division associées à un traitement par le ganciclovir conduit à la mort de la cellule qui le contient suite à une incorporation de l'analogue de nucleoside dans l'ADN des cellules et un arrêt subséquent de la réplication. L'avantage de l'incorporation de ce gène dans le plasmovirus est de stopper le cas échéant une dissémination virale voulue ou accidentelle.
   Tout dérivé de la thymidine kinase qui conserve la fonction phosphorilante de cette dernière peut être utilisé, c'est le cas par exemple des dérivés tronqués tels que décrits dans la demande de brevet n° PCT/FR/95/01098. De façon plus générale, tout gène suicide ou même tout gène dont le produit est susceptible d'agir sur la cellule hôte ou le virus sous l'action d'une substance exogène audit virus peut être de la même façon intégré dans le vecteur.
b) des gènes codant pour des immunomodulateurs. Il peut s'agir par exemple :
   - de gènes de cytokine : lorsque le plasmovirus est injecté par voie intraveineuse, un plasmovirus qui n'aurait pas transfecté des cellules et permis la reconstitution d'une particule physique infectieuse serait alors capté par le système réticuloendotélial et la présence de gènes codant pour des cytokines, en particulier des interleukines ou le G-CSF, GM-CSF ;
   - ou une protéine du CMH (complexe majeur d'histocompatibilité) devrait favoriser la présentation antigénique ;
   - ou de molécule de co-stimulation comme B7-1 ou B7-2 (Jenkins MK et al, 1993, Curr. Opin. immunol. 5 : 361-367).
Le produit d'un tel gène secrété localement peut également induire en tant que tel une stimulation de la réponse immunitaire.
c) des gènes ou séquences nucléotidiques codant pour des peptides, polypeptides ou protéines pour lesquels on souhaite déclencher ou augmenter une immunogénicité : il peut s'agir alors, par exemple, d'haptènes ou d'épitope d'antigène contre lesquels on cherche à développer une immunisation ; la particule virale reconstituée à partir du plasmovirus joue en quelque sorte un rôle d'adjuvant ou de transporteur conférant à l'antigène ou l'épitope particulier une immunogénicité. Dans ce dernier cas, il est particulièrement intéressant d'insérer la séquence complémentaire dans une région du gène env qui permet de garder la fonctionnalité de la partie extra-membranaire de la protéine d'enveloppe tout en ayant acquis la structure de l'épitope particulier exprimée en surface de la particule virale.

Dans tous les cas, c'est la particule physique reconstituée in vivo qui agit comme un vaccin élicitant une réponse cellulaire et humorale. Cette approche est particulièrement intéressante dans le cas de la recherche d'un vaccin protecteur contre un virus pour lequel aucun vaccin n'a pu être mis au point. C'est le cas notamment des HIV et des HTLV, spumavirus ou rétrovirus de type D, pour lesquels toutes les tentatives de mise au point de vaccins se sont révélées insuffisantes quant à leur effet protecteur (Médecine / Science, 1996 n° 1 volume 12, pages 87-93). Ainsi, dans la présente invention, le vaccin est particulièrement intéressant lorsque le génome viral du plasmovirus est celui d'un rétrovirus, et que le vecteur codant contient au moins un gène de structure HIV.

Les propriétés des plasmovirus, et donc des vaccins de l'invention, résultent également du type d'enveloppe utilisé. En effet, la protéine d'enveloppe intervient non seulement dans l'efficacité de production de particules matures infectieuses mais aussi permet de conférer à cette particule une spécificité d'infection (tropisme) car c'est cette protéine qui intervient dans les phénomènes de liaison du virion à la cellule par l'intermédiaire de récepteurs membranaires, et elle est impliquée dans la fusion des membranes virales et plasmiques, qui permet la libération de la nucléocapside dans le cytoplasme des cellules infectées. Enfin, l'enveloppe joue un rôle important dans la résistance du vecteur à l'inactivation par le complément. De cette façon, sur une charpente nucléocapsidique, on peut modifier le spectre d'infection du vecteur en modifiant le gène de l'enveloppe, permettant ainsi de l'adapter à la stratégie de vaccination que l'on souhaite adopter.

A titre d'exemple, il a pu être constaté que des vecteurs comportant le gène env du VSV (virus de la stomatique vésiculaire) ont un large tropisme vis-à-vis des cellules cibles, ce caractère de type ubiquitaire résultant de la nature du récepteur membranaire de cette enveloppe. Au contraire, des gènes env provenant de virus écotropes auront une spécificité beaucoup plus étroite.

Un gène ou un fragment de gène codant pour un peptide, un polypeptide ou une protéine peut être inséré également dans la partie N-terminale du gène env, comme cela a été décrit par VALSESIA-WITTMANN et al, Journal, of Virology,(1996), pp 2059-2064, et ce sans interférer avec la liaison du virion avec son récepteur sur les cellules cibles. A l'occasion de la reconstitution de la particule virale, le peptide ou le polypeptide résultant de la traduction du gène ou du fragment inséré va être associé de façon covalente à la partie N-terminale de l'enveloppe virale, c'est-à-dire dans la partie extra-membranaire de la particule. Cette propriété peut être mise avantageusement à profit pour conférer à la particule reconstituée à partir du plasmovirus, différentes propriétés qui peuvent notamment être les suivantes :
a) déclenchement d'une réaction immunitaire à médiation cellulaire.
   Quand le peptide, le polypeptide ou protéine -que nous appelerons globalement "insert"-, est exprimé en surface de la molécule virale reconstituée, cela peut conduire à une stimulation de l'immunité à médiation cellulaire par activation des cellules T et simultanément activation des macrophages ou des cellules B productrices d'anticorps. Cette capacité peut ainsi être mise à profit pour augmenter l'immunogénicité de la particule virale ainsi reconstituée. Quand l'insert est un antigène vaccinant, la particule reconstituée à partir du plasmovirus peut alors être un adjuvant qui augmente en tant que tel la réponse immune à l'antigène exprimé à la surface de la particule.
b) déclenchement d'une réponse immunitaire vis-à-vis d'un haptène :
   Un haptène est un déterminant antigénique dépourvu par lui-même de pouvoir immunogène. Une réaction antigénique contre des haptènes est déclenchée et/ou amplifiée par couplage de l'haptène à un transporteur ("carrier") qui peut, par exemple, être de l'albumine, de la PPD (purified protein derivatives of tuberculin) ou la KLH. Généralement, le porteur est reconnu par les lymphocytes T, alors que l'haptène interagit avec les cellules de type B. Sont alors de bons candidats comme "inserts" les haptènes polypeptidiques.
c) fonction de routage :
   Quand "l'insert" est choisi pour son affinité vis-à-vis d'un récepteur spécifique d'une cellule cible, que celle-ci soit insérée ou isolée dans un organe, la particule virale exprimant cet "insert" peut être alors dirigée de préférence sur la cible donnée pour y déclencher spécifiquement son action. Si l'action est simplement le déclenchement d'une réaction d'une immunité cellulaire ou humorale, le routage peut être effectué directement sur les cellules T. Si, en revanche, un transgène est inséré dans le plasmide comme cela est représenté, par exemple, sur la figure 2, le routage permet de cibler spécifiquement le gène en question dans la cellule dans laquelle il doit s'exprimer pour obtenir une immunogénicité optimale à partir du moment où la particule virale a des propriétés d'infectivité vis-à-vis de cette cellule cible.
d) les trois propriétés décrites plus haut ne sont bien entendu pas exclusives l'une de l'autre et peuvent être complémentaires pour en faire des particules vaccinantes présentant une immunogénicité et/ou une spécificité accrue.

De la même façon, il a été montré qu'une protéine du virus herpès-simplex de type 1 (HSV-1) appelée Us11 est capable d'activer en trans l'expression de la glycoprotéine d'enveloppe du rétrovirus et notamment de HIV ou du HTLV (J. Diaz et al. (1996), Nature 379 : 273). L'inclusion du gène codant pour Us11 dans le plasmovirus soit sous la dépendance du promoteur du rétrovirus, soit sous la dépendance d'un autre promoteur, par exemple le promoteur pCMV utilisé pour la transcription d'un gène de structure du rétrovirus est particulièrement intéressante.

Aussi, et selon le gène ou le fragment de gène que l'on souhaite exprimer, les cellules cibles que l'on souhaite viser, le gène env et le lieu d'insertion pourront être déterminés.

Un premier exemple de construction de plasmovirus est représenté dans la figure 1. Il comprend :
a) les gènes gag et pol sous le contrôle d'un promoteur spécifique ;
b) un gène env également sous le contrôle d'un promoteur spécifique ;
c) un signal de polyadenylation différent du LTR du virus considéré, de type SV40, TK, HBV,...
d) le cas échéant la séquence Ψ,
e) le cas échéant un gène ou fragment de gène d'intérêt dont l'expression dépend soit du promoteur en a) soit du promoteur en b) soit sur une cassette d'expression indépendante.

Dans cette construction, toute reconstitution d'un génome viral est impossible en l'absence des LTR. Cette stratégie est appropriée pour des virus « simples » ayant peu de cadres de lectures. Dans d'autres versions, chacune des protéines nécessaires peut être placée dans une cassette d'expression indépendante. Pour des virus complexes comme le VIH, plusieurs cassettes peuvent se succéder (gag/pol, env, tat, rev, nef).

Un autre exemple de plasmovirus selon l'invention est représenté sur la figure 2 et peut comprendre :
a) le gène env d'un rétrovirus sous le contrôle d'un promoteur spécifique,
b) un génome rétroviral défectif, comprenant les séquences LTR, les gènes gag et pol, le cas échéant la séquence Psi.
c) le cas échéant un gène ou fragment de gène d'intérêt dont l'expression dépend soit du promoteur en a) soit du promoteur en b),
d) le cas échéant le gène Us11 de HSV ;

Cette construction voisine de celle des plasmovirus déjà décrits dans l'état de la technique permet l'expression par la cellule transduite d'un gène étranger qui peut être par exemple un gène codant pour une protéine immunostimulante. De plus dans une version Ψ⁺ de cette construction, des particules infectieuse mais défectives sont produites. Ainsi des cellules peuvent être infectées et exprimer de nouveau les protéines nécessaires à la formation de particules virales. Si ces cellules sont des cellules présentatrices de l'antigène, ceci peut être particulièrement avantageux.

Un autre mode de réalisation d'un vecteur selon l'invention peut comprendre :
a) les gènes gag, pol et/ou env d'un rétrovirus sous le contrôle d'un promoteur spécifique,
b) un génome rétroviral défectif, comprenant les séquences LTR, la séquence Psi,
c) le cas échéant un gène ou fragment de gène d'intérêt dont l'expression dépend soit du promoteur en a) soit du promoteur en b),
d) le cas échéant le gène Us11 de HSV.

Un autre exemple de plasmovirus est représenté sur la figure 3 et comprend un génome viral atténué, dépourvu de séquence Ψ et dont plusieurs remaniements sont introduits dans le génome pour éviter l'apparition de virus réplicatifs. Le LTR 3' est remplacé par un site de polyadénylation différent ; des mutations (mutations ponctuelles, délétions, changement de phase de lectures...) sont réalisées dans une ou plusieurs protéines non structurale (transcriptase inverse RT, intégrase IN). Cette stratégie est particulièrement appropriée pour des génomes complexes comme HIV qui possèdent un grand nombre de protéines ; leur synthèse qui est complexe reste ainsi sous le contrôle homologue du LTR 5' du HIV.

Pour toutes les constructions (figures 1, 2, 3), on peut rajouter sur le plasmide une ou plusieurs cassettes d'expression codant pour des gènes favorisant la réponse immunitaire. De plus, les éléments favorisant l'expression du plasmide peuvent être également ajoutés : ITR de AAV, séquences pouvant favoriser la réplication du plasmide (Origine de réplication de EBV ou SV40 et cassette d'expression ebna ou antigène T par exemple)...

La présente invention porte également sur une composition immunogène, notamment un vaccin, pour la prophylaxie et/ou la thérapeutique d'individus infectés ou susceptible d'être infectés par un virus, et contenant au moins un plasmovirus tel que défini ci-dessus.

Les sujets concernés sont les humains et les animaux, et les compositions immunogènes ou vaccins peuvent relever de la médecine humaine ou vétérinaire.

Cette composition est immunogène plus particulièrement dans le sens où l'injection du vecteur porteur du génome viral défectif permet la reconstitution d'une particule physique susceptible d'éliciter une réponse immunitaire cellulaire et/ou humorale.

Le génome viral défectif et les séquences codant pour le ou les gène(s) permettant la reconstitution de l'enveloppe virale peuvent être portés par un ou deux vecteurs, qui sont associés dans la composition, ceux-ci peuvent être liés par des moyens connus de l'homme du métier, notamment ceux décrits dans la demande de brevet WO 95/26411. Dans ce cas, le terme de plasmovirus s'applique aussi bien à un vecteur unique portant aussi bien les séquences spécifiques du génome viral défectif, et les séquences codant pour la ou les protéine(s) de structure ainsi que le cas échéant les séquences codant pour les gènes exogènes cités ci-dessus, soit pour augmenter le pouvoir immunogène, soit pour augmenter la sécurité du système, soit enfin pour permettre une meilleure expression des protéines de l'enveloppe virale.

La composition immunogène peut également contenir à titre de principe actif des cellules qui ont été préalablement transfectées in vitro par un plasmovirus ou un vecteur tels que décrits ci-dessus. Dans ce cas, une injection de la composition permet la greffe de la cellule hôte in vivo, et la reconstitution d'une particule virale qui est alors susceptible de déclencher la réponse immunitaire recherchée.

La composition immunogène peut, le cas échéant, contenir associée au plasmovirus un adjuvant de vaccination. Les adjuvants peuvent être de plusieurs types ; certains favorisent la réponse immunitaire classique comme un adjuvant de type N-acétyl-muramyl associé à un peptide tel le MDP ou ses différents dérivés tels que ceux décrits dans.CRS Critical Reviews in Immunology, 1988, Vol. 8, pages 83-100. D'autres types d'adjuvant peuvent être envisagés comme ceux qui favorisent la pénétration des séquences d'acides nucléiques dans les cellules ; enfin, le plasmovirus dans la composition immunogène peut être lié à une séquence connue comme ou présumée T-dépendante ou alternativement liée ou englobée dans des liposomes. Il peut être aussi administré dans des milieux aqueux ou huileux, sous forme d'injections sous-cutanée, intramusculaire ou intraveineuse par « pellets » (polymères biodégradables contenant le produit) et/ ou par implantation du système de relargage par micropompe. D'autres adjuvants, tels que le PAO, seuls ou associés à la lécithine (EP445-710) ZN(OH) ou HBW538 (Drugs experimental clinical research (1991) 17 (9) : 445450) associés à l'hydroxyde d'aluminium (Al(OH) 3) peuvent être associés de façon covalente ou non au principe actif pour la composition. La composition immunogène dont l'un des principes actifs est le plasmovirus est également caractérisée dans le fait que génome viral défectif est porteur d'un gène suicide afin de permettre la sécurité du système par addition le cas échéant de ganciclovir lorsque l'on souhaite arrêter la prolifération dudit virus.

Dans la composition immunogène de l'invention le plasmovirus peut être, soit de l'ADN nu ou enrobé, soit de l'ADN nu associé à une cellule de type cellule d'empaquetage ou encore une cellule établie en lignée qui peut être choisie par exemple parmi les lignées fibroblastiques comme la lignée 3T3 ou des lignées susceptibles d'être cultivées en suspension comme les cellules de myélome, les cellules Véro ou les cellules MRC5. Dans ce cas, la composition immunogène est l'ensemble du plasmovirus et de la cellule hôte qui agit, dans ce cas-là, comme une greffe cellulaire permettant de produire la particule virale qui, elle-même, permettra d'éliciter la réaction vaccinale.

La composition immunogène de l'invention est caractérisée en ce qu'elle contient de 0.1 à 1000 µg d'acides nucléiques constitutifs dudit vecteur par dose et de préférence de 10 à 500 µg par dose. Ces doses s'entendent par dose unitaire destinée à des humains.

Dans la composition immunogène de l'invention, le plasmovirus peut également être un virus tel l'adénovirus ou l'AAV (adenoassociated virus) défectif et porteur de toutes les séquences nécessaires à la reconstitution d'un rétrovirion ; autrement dit, le virus défectif, porteur des séquences rétrovirales joue le rôle de transporteur vis-à-vis de cellules qui doivent être ciblées de façon préférentielle.

A titre d'exemple, l'AAV a un tropisme particulier pour les cellules musculaires qui ont une forte capacité immunogène ; l'introduction du génome porteur des séquences rétrovirales dans ce type de cellules peut en augmenter l'effet vaccinant.

Dans ce mode de réalisation, la composition de plasmovirus contient de 100 à 10⁶ particules virales défectives par dose unitaire destinée de préférence à des humains.

La présente invention porte également sur une méthode d'immunisation active l'individu contre une infection virale, ladite méthode consistant à injecter à un individu un plasmovirus porteur des gènes de structure nécessaires à la reconstitution d'une particule virale.

Le plasmovirus injecté peut porter en outre un génome viral ou rétroviral, de préférence défectif et porteur de séquences non codantes et/ou régulatrices du virus ou du rétrovirus, et le cas échéant d'une séquence d'encapsidation. Les génomes rétroviraux qui sont préférés dans le cadre d'une méthode de vaccination sont ceux des lentivirus, oncornavirus, spumavirus ou de rétrovirus de type D. Dans ce cas également, les gènes de structure nécessaires à la constitution de la particule virale sont de préférence les gènes codant pour gag et/ou env. Le gène env. est choisi en fonction du but assigné au plasmovirus, différents exemples étant cités plus haut. Le gène env. peut être recombiné de telle façon qu'une chimère soit produite, qui porte à la fois la fonction d'attachement au récepteur des cellules cibles du virion et du recepteur de l'insert dans la molécule env. Comme il était dit plus haut, il peut être avantageux également d'adjoindre au plasmovirus le gène Us11 de HSV1 de façon à stimuler la synthèse des protéines de structure.

Les différents modes de réalisation des vecteurs utilisés dans la vaccination ont été décrits ci-dessus.

La méthode de vaccination peut consister également à traiter l'individu infecté ou susceptible d'être infecté par une cellule hôte préalablement transfectée par les vecteurs de l'invention et notamment les lignées fibroplastiques de type 3T3 ou des lignées cultivées en suspension comme les cellules de myelome ou les cellules Vero.

Lorsque génome rétroviral est dépourvu de la séquence d'encapsidation, les particules physiques reconstituées sont alors dépourvues de génome. Au contraire quand le génome viral est porteur de cette séquence d'encapsidation, il peut être intéressant de construire le vecteur de telle façon que sous la dépendance du promoteur rétroviral, un gène d'intérêt soit ajouté de façon à augmenter la sécurité du système ou à augmenter la réponse immunitaire du vaccin.

Cette immunisation peut être obtenue après injection percutanée ou sous-cutanée, intramusculaire ou intraveineuse d'une composition immunogène telle que décrite ci-dessus. Ces plasmovirus peuvent être également utilisés dans la fabrication de la composition immunogène et notamment d'un vaccin afin de prévenir ou traiter les infections virales, cette utilisation faisant partie de l'invention.

L'utilisation de plasmovirus est avantageuse dans la mesure où la préparation et la conservation d'acides nucléiques est plus aisée à mettre en oeuvre que la production et la conservation de particules virales complètes.

La formation de particules virales défectives peut être induite soit in vitro dans des cellules en culture, soit in vivo après injection intramusculaire, intraveineuse ou sous-cutanée de la composition immunogène, soit ex-vivo après injection des cellules transformées.

Enfin, le procédé de fabrication d'un vaccin vivant constitué d'une particule virale reconstituée, de préférence défective, fait partie de l'invention ; il comprend la transfection de cellules par un vecteur tel que décrit ci-dessus comportant, d'une part, le gène de structure nécessaire à la reconstitution de la particule virale, d'autre part, le génome rétroviral défectif ; les gènes de structure du virus étant, soit sous la dépendance d'un promoteur spécifique, soit sous la dépendance de promoteur viral.

Les cellules transfectées dans le procédé de l'invention sont, soit des cellules en culture et la transformation est effectuée in vitro, soit les cellules sont celles d'un individu et sont transfectées in vivo ou ex vivo.

Ce procédé est particulièrement intéressant sur le plan économique d'une part et de la sécurité d'autre part. En effet, la production des plasmovirus est simple, et la stabilité et la reproductibilité mieux en accord avec les impératifs de production de médicaments. Par ailleurs, cela permet d'éviter la manipulation de particules virales qui, à une étape d'un procédé de préparation, par exemple de vaccins vivants par virus entiers atténués ou inactivés, pourrait être dangereux.

L'homme du métier saura utiliser au cas par cas les techniques les plus appropriées à la transfection de tel ou tel type cellulaire. Les exemples ci-dessous ainsi que les schémas dont les légendes suivent servent à illustrer l'invention sans limiter son étendue.

L'exemple ci-après ainsi que l'exemple comparatif montrent l'efficacité de l'approche vaccinale sur un modèle animal.

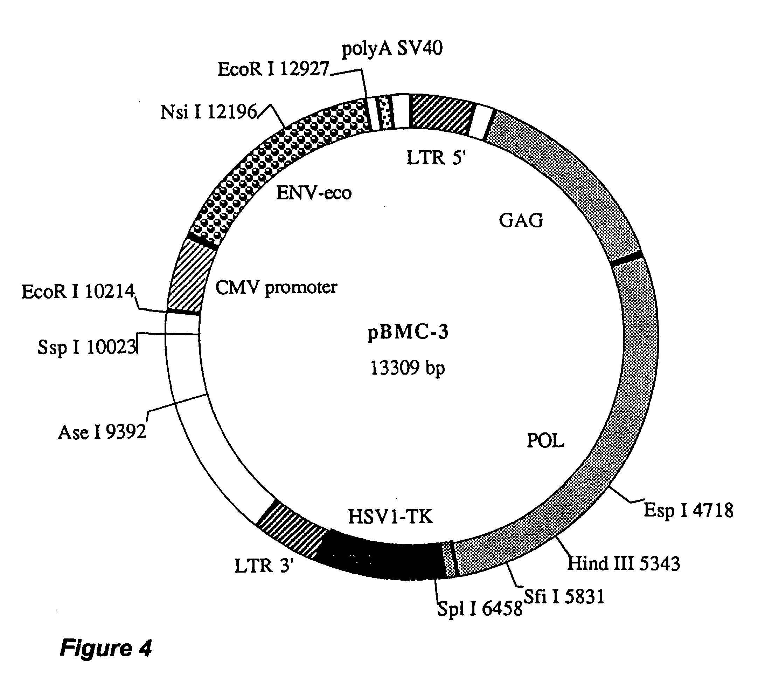

Dans cet exemple, la figure 4 représente le plasmide pBMC-3 comportant un gène HSV1-TK sous le contrôle d'un promoteur LTR 5' Mo-MuLV.

La figure 5 représente le plasmide pNP-2.

La figure 6 représente la variation de poids des animaux vaccinés à l'aide de ces constructions et infectés par le virus de la leucémie myéloproliférative murine. En abscisse sont indiqués les jours d'examen et en ordonnée les variations des poids des animaux.

### I. Modèle expérimental utilisé :

Le modèle expérimental utilisé est l'induction d'une leucémie mégacaryocytaire chez la souris. Cette leucémie est induite par infection de la souris par un rétrovirus pathogène de type MPLV. Le virus MPLV est dérivé des virus de leucémies murines de type friend. Ces derniers ont des séquences LTR distinctes des virus de type moloney mais ont des séquences de protéine de structure extrêmement voisines. Il est donc possible de vacciner contre le MPLV en utilisant des protéines dérivées des virus de moloney.

Un tel virus porte un oncogène v-mpl, qui est le ligand de la thrompoïétine. Après infection par un tel virus, la souris développe en quelques semaines la leucémie mégacaryotique. Cette maladie est caractérisée par une spléno-mégalie et une hépatomégalie; plus précisément, après infection virale, un nombre extrêmement important de lymphocytes s'accumulent dans la rate. Aussi un effet vaccinant peut-il être apprécié par l'absence d'une augmentation du poids de la rate, du foie ou de l'animal entier après un challenge avec du virus pathogène.

### II. Plasmovirus utilisés dans ce modèle :

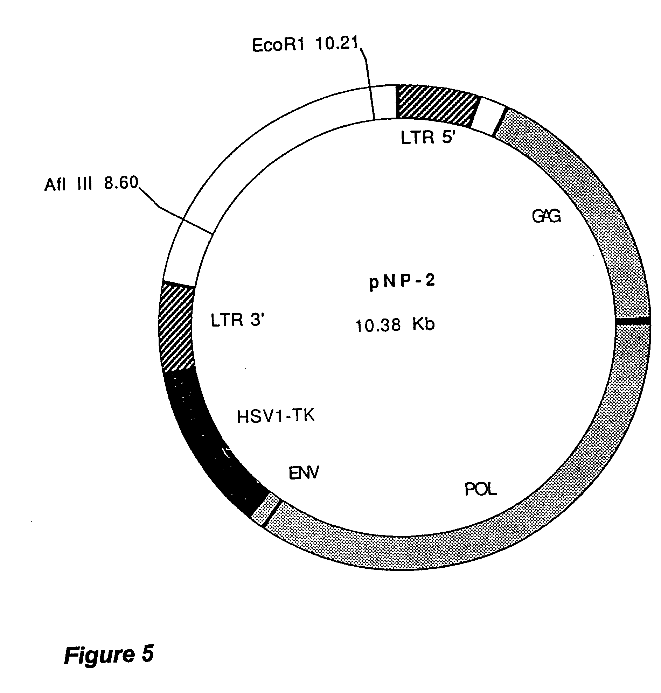

La vaccination a été effectuée par trois plasmovirus différents qui sont les suivants :
- Le plasmide pNP-2, représenté sur la figure 5, est un plasmide de 10 380 paires de bases. Dans ce plasmide, le premier codon ATG du gène env d'un provirus dérivé de moloney a subi une mutation ponctuelle afin de garder le sens du codon correspondant dans le gène pol qui se superpose. Quelques paires de bases en aval du codon stop du gène pol, un gène HSV1-TK a été inséré de façon à enlever toute séquence du gène env entre le codon stop de ce transgène et la fraction polypurine du LTR en 3'. Dans une telle construction, les gènes gag et pol sont obtenus à partir d'un RNA génomique non coupé alors que le transgène est transcrit à partir d'un RNA épicé en utilisant des sites donneurs et accepteurs d'épissage du provirus sauvage. Une cassette d'expression du gène env a ensuite été construite de façon à réduire la possibilité de recombinaison conduisant à des virus infectieux ;
pBMC-3, représenté dans la figure 4, est le même que le plasmide pNP-2 mais comprend en plus le gène env. écotropique ;

Le plasmide pBPXT4 incorporant le gène humain du CD4 et dépourvu du gène env, non représenté ici, a été utilisé comme témoin.

### III. Effet prophylactique protecteur des plasmovirus dans le modèle animal :

Des souris adultes sont injectées par voie intramusculaire tel que décrite par Ulmer et al, Science, 1993 259 : 1745-1749.

Les injections à raison de 100µg de plasmide préparé par Qiagen par souris sont répétées trois fois à une semaines d'intervalle.

Comme groupe de contrôle de l'efficacité de la vaccination, les souris sont injectées par un ADN plasmidique totalement étranger au virus MPLV.

A l'issue de la procédure d'immunisation, les animaux sont injectés par voie intraveineuse par un surnageant de culture d'une lignée cellulaire produisant le virus MPLV.

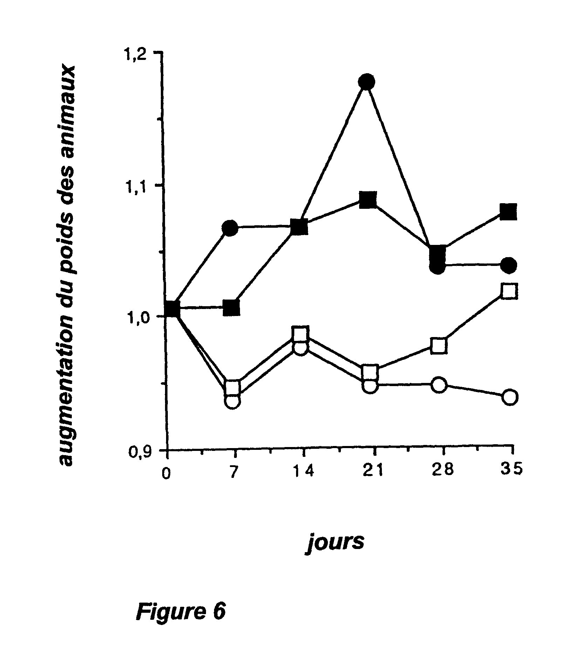

Ce surnageant non dilué a une activité de reverse transcriptase (RT) de 66 152 CPM/10⁶ cellules. Ce surnageant a été dilué 100 fois et 200 microlitres ont été injectés aux souris par voie intraveineuse et le moment de l'injection correspond au temps 0 de la courbe de la figure 6.

Quatre groupes d'animaux ont été traités ; chaque groupe consistant en cinq souris dBA/2 de dix semaines au début de l'expérimentation, c'est-à-dire à la première vaccination.

A différents temps après l'injection des particules du MPLV, les animaux sont soit pesés lorsqu'ils sont encore vivants et/ou sacrifiés et le poids de la rate et du foie respectivement pesé. Les temps de mesure et de prélevement sont effectués à 7, 14, 21, 28 et 35 jours.

### IV. Résultats :

La figure 6 représente la variation du poids de l'animal à différents temps après le challenge par les particules du MPLV. Les ronds blancs représentent les résultats obtenus après une vaccination par le plasmide pBMC-3. Le rond noir représente les résultats obtenus après vaccination par pNP-2. Le carré blanc représente les résultats obtenus avec pBXT4, plasmide apportant le cDNA du CD4 humain, et les carrés noirs représentent le témoin dans lequel les souris n'ont pas été vaccinées préalablement à l'injection des particules virales.

Le tableau ci-dessous indique respectivement le poids de la rate et du foie après prélèvement, 21 jours après l'injection des particules virales.

| Type de vaccination | Accroissement du poids de la rate au 21è jour | Accroissement du poids du foie au 21è jour |
|---|---|---|
| pBMC-3 | x 1 | x 0,8 |
| pNP-2 | x 16,3 | x 1,3 |
| hCD4 | x 12,5 | x 1,3 |
| aucun vaccin | x 11,0 | x 1,1 |

### Conclusion :

Les résultats montrent clairement que la vaccination par pBMC-3 est efficace dans la mesure où aucune augmentation de poids n'a été constatée ni chez la souris ni sur la rate ni sur le foie. Au contraire, l'utilisation des trois autres plasmovirus conduit à une augmentation d'au moins dix fois du poids de la rate indiquant le développement de la maladie et une inefficacité de la vaccination. Ce résultat montre clairement que le plasmovirus intégré dans une composition, donne naissance à un principe actif capable de prévenir l'effet pathogène d'une infection virale.

## Revendications

1. Vaccin constitué de particules virales défectives telles qu'obtenues in vivo ou ex vivo chez des vertébrés infectés ou susceptibles d'être infectés par un virus, après transfection des gènes portés par un plasmovirus constitué d'un vecteur ou une association de vecteurs et comprenant au moins les gènes de structure nécessaires à la reconstitution d'une particule virale porteuse de déterminants immunogènes.

2. Vaccin selon la revendication 1 caractérisé en ce que les particules virales constituées sont rétrovirales, et le vecteur comprend au moins le gène gag et le gène env du retrovirus correspondant.

3. Vaccin selon l'une des revendications 1 à 2 caractérisé en ce que le vecteur contient en outre le gène Us11 de HSV₁.

4. Vaccin selon l'une des revendications 1 à 3 caractérisé en ce que le vecteur comprend:
a) gag ou gag/pol sous le contrôle d'un promoteur spécifique,
b) un gène env également sous le contrôle d'un promoteur spécifique,
c) un signal de polyadenylation différent du LTR du virus considéré, type SV40, TK, HBV,...
d) le cas échéant la séquence Ψ,
e) le cas échéant un gène ou fragment de gène d'intérêt, soit dont l'expression dépend soit du promoteur en a) ou du promoteur en b), soit sur une cassette d'expression indépendante.

5. Vaccin selon l'une des revendications 1 à 3 caractérisé en ce que le vecteur comprend:
a) les gènes gag, pol et/ou env d'un rétrovirus sous le contrôle d'un promoteur spécifique,
b) un génome rétroviral défectif, comprenant les séquences LTR, la séquence Psi,
c) le cas échéant un gène ou fragment de gène d'intérêt dont l'expression dépend soit du promoteur en a) soit du promoteur en b),
d) le cas échéant le gène Us11 de HSV.

6. Vaccin selon l'une des revendications 1 à 4 caractérisé en ce que le vecteur comprend un génome rétroviral, dépourvu de la séquence Ψ, et dans lequel des mutations sont réalisées dans les gènes codant pour les protéines non structurales, telles pol ou INT.

7. Vaccin selon la revendication 4 à 6 caractérisé en ce que le gène d'intérêt code pour une toxine conditionnelle comme la Thymidine Kinase de HSV1-TK.

8. Vaccin selon la revendication 4 à 6 caractérisé en ce que le gène ou fragment d'intérêt est un gène capable de stimuler la réponse immunitaire, notamment un gène de cytokine, du CMH ou la molécule B7.

9. Vaccin selon la revendication 4 à 6 caractérisé en ce que le gène ou fragment d'intérêt est intégré dans le gène env.

10. Vaccin selon la revendication 9 caractérisé en ce que le gène ou fragment d'intérêt code pour un haptène polypeptidique.

11. Vaccin selon l'une quelconque des revendications précédentes caractérisé en ce que le virus est un rétrovirus choisi parmi les lentivirus, oncornavirus, Spumavirus ou un rétrovirus de type D.

12. Vaccin selon l'une quelconque des revendications précédentes dans lequel une séquence codant pour un peptide, polypeptide ou protéine est insérée à l'extrémité 5' de la séquence env.

13. Vecteur susceptible d'être utilisé dans la production d'un vaccin selon la revendication 1 et comprenant :
a) le gène env d'un rétrovirus sous le controle d'un promoteur spécifique,
b) un génome rétroviral défectif, comprenant les séquences LTR, les gènes gag et pol, et le cas échéant la séquence Ψ,
c) le cas échéant un gène ou fragment d'intérêt, soit dont l'expression dépend soit du promoteur en a) ou du promoteur en b), soit sur une cassette d'expression indépendante,
d) le cas échéant le gène Us11 de HSV.

14. Vecteur susceptible d'être utilisé dans la production d'un vaccin selon la revendication 1 et comprenant :
a) les gènes gag, pol et/ou env d'un rétrovirus sous le contrôle d'un promoteur spécifique,
b) un génome rétroviral défectif, comprenant les séquences LTR, avec ou sans la séquence Ψ,
c) le cas échéant un gène, soit dont l'expression dépend soit du promoteur en a) ou du promoteur en b), soit sur une cassette d'expression indépendante,
d) le cas échéant le gène Us11 de HSV.

15. Vecteur selon la revendication 13 ou 14 caractérisé en ce qu'une séquence codant pour un peptide, polypeptide ou protéine est inséré dans le gène env.

16. Vecteur selon les revendications 13 à 14 caractérisé en ce que le gène d'intérêt est celui de la Thymidine Kinase de HSV1-TK.

17. Vecteur selon les revendications 13 à 14 caractérisé en ce que le gène d'intérêt est un gène capable de stimuler la réponse immunitaire, notamment un gène de cytokine, du CMH ou une protéine B7.

18. Composition immunogène pour la prophylaxie et/ou la thérapeutique d'individus infectés ou susceptibles d'être infectés par un virus caractérisée en ce qu'elle contient au moins un vecteur selon l'une des revendications 13 à 17.

19. Composition immunogène selon la revendication 17 ou 18 caractérisée en ce que le virus est un rétrovirus, notamment un HIV ou un HTLV, un Spumavirus, un rétrovirus de type D.

20. Composition immunogène selon l'une des revendications 18 ou 19 caractérisée en ce qu'elle contient 0,1 à 1000 µg par dose d'acide nucléique constitutif dudit vecteur.

21. Composition immunogène selon l'une des revendications 18 à 20 caractérisée en ce qu'elle constitue un vaccin, notamment contre les rétrovirus.

22. Utilisation dans la fabrication d'une composition immunogène et notamment d'un vaccin, destinée à prévenir ou traiter les infection virales, d'un vecteur susceptible d'induire la formation d'une particule virale in vivo ou ex vivo.

23. Utilisation selon la revendication 22 caractérisée en ce que les infections virales sont des infections rétrovirales.

24. Utilisation selon la revendication 23 caractérisée en ce que la composition immunogène est un vaccin contre un HIV, un HTLV, un spumavirus ou un rétrovirus de type D.

25. Procédé de fabrication d'un vaccin vivant constitué d'une particule virale défective, comprenant la transfection de cellules in vivo ou ex vivo par un vecteur selon l'une des revendications 11 à 16.

26. Procédé selon la revendication 25 caractérisé en ce que la particule virale défective est une particule rétrovirale.

27. Utilisation d'un vecteur selon l'une des revendications 13 ou 14 dans la fabrication d'une composition capable d'induire une immunisation active contre des haptènes.

28. Procédé de ciblage d'une composition immunogène caractérisé en ce qu'il comprend l'insertion dans le gène env. d'un vecteur selon l'une des revendications 13 ou 17 d'une séquence codant pour un peptide ou polypeptide ayant une affinité spécifique pour un récepteur cellulaire.
